# EUROPEAN PATENT SPECIFICATION

(11) **EP 0 231 605 B1**
(45) Date of publication and mention of the grant of the patent: **07.04.1993**
(21) Application number: 86309249.0
(22) Date of filing: 27.11.1986
(51) Int. Cl.: C12P 25/00, C12N 15/04

(54) **Riboflavin production with yeast**
Herstellung von Riboflavin mit Hefe
Production de riboflavine avec de la levure

(30) Priority: 20.12.1985 US 811234
(43) Date of publication of application: 12.08.1987
(73) Proprietor: ZeaGen, Inc., Broomfield, CO 80021 (US)
(72) Inventor: Heefner, Donald L., Longmont Colorado 80501 (US); Yarus, Michael, Boulder Colorado 80302 (US); Boyts, Annette, Golden Colorado 80403 (US); Burdzinski, Linda, Louisville Colorado 80027 (US)
(74) Representative: Harrison, David Christopher

(56) References cited:
- EP-A- 0 137 226
- BE-A- 890 917
- FR-A- 2 364 968
- CHEMICAL ABSTRACTS, vol. 86, part 11, 14th March 1977, page 272, abstract no. 68254s, Columbus, Ohio, US; A.A. IMSHENETSKII et al.: "Variability of polyploid strains of candida scottii in accumulation of riboflavin in the medium", & Z. ALLG. MIKROBIOL. 1977, 17(1), 7-10

## Description

### 1.

The present invention relates to the generation and isolation of flavinogenic strains of Candida flareri for the production of riboflavin. During the pendency of this application, the taxonomic classification of this organism has been changed to C. famata : however the previous terminology has been retained herein.

Riboflavin, most commonly known as vitamin B₂, an enzyme cofactor is found to some degree in virtually all naturally occurring foods. It is widely used in pharmaceuticals, food-enrichment, and feed supplements.

Riboflavin has been commercially produced by fermentation. Most commercially produced riboflavin is consumed as a crude concentrate for enriching animal feed. Riboflavin also finds use for human consumption. The present invention is concerned with the development of Candida flareri strains for the efficient and economic production of riboflavin.

### 2.

Varieties of Candida yeasts are attractive as flavinogenic organisms because they grow rapidly on a simple, well known medium. P.R. Burkholder, Proc. Nat. Acad. Sci., U.S.A., (1943) 29:166.

Candida flareri has been studied for a number of years for its flavinogenic capacities. See, e.g., Enari and Kauppinen, Acta. Chem. Scand. - (1961) 15:1513. In particular, the inhibitory effect of iron and trace elements on riboflavin production has been investigated. Tanner et al., Science (1945) 101:180. So far, riboflavin production by C. flareri has proven to be highly variable. Early pilot plant fermentations using simple non-sterilized culture mediums gave riboflavin yields of 325mg/liter. Levine, H., et al., Industrial and Engineering Chemistry (1949) 41:1665.

Certain varieties of Candida yeasts have been known to produce substantial amounts of riboflavin. For example, a modification employing Candida intermedia var A, a newly isolated microorganism assimilating lactose and ethanol, has been disclosed in Japanese Patent No. 73/19,958 and yields reported of 49.2 mg riboflavin per liter. Candida T-3 also produces riboflavin from methanol, as disclosed in Japanese Patent No. 76/19,187. See also, EPA 0 137 226 for disclosure of Candida robusta for riboflavin production.

In the invention, enhanced riboflavin production is achieved employing a mutated Candida strain. Starting with a riboflavin producing Candida strain, the strain is subjected to a regimen of chemical and/or physical mutagenesis and protoplast fusions of mutagenized strains to produce a strain having efficient production of riboflavin and reduced sensitivity to iron and inhibitors of purine and riboflavin biosynthesis. A mutant, dgr, was also isolated and exhibited enhanced flavinogenic activity.

Methods and organisms are provided for enhanced production of riboflavin. A riboflavin-producing Candida strain is selected, subjected to a course of mutagenesis, mutagenized strains screened as to a variety of traits associated with flavinogenesis, desirable mutants are fused, the fusion progeny screened and subjected to further mutagenesis to provide strains producing riboflavin with high efficiency at stable and reproducible levels, with reduced resistance to repression of the B₂ pathway by resistance to catabolite repression by glucose, resistance to inhibition of the purine pathway by adenosine monophosphate, and the adenine anti-metabolite 4-amino pyrazolo(3,4-d)-pyrimidine (APP).

Mutants are selected where the riboflavin synthesis is no longer as sensitive to or insensitive to flavin adenine dinucleotide and/or adenosine monophosphate regulation, where phosphoribosyl- pyrophosphate amidotransferase has reduced sensitivity to regulation by adenine and adenosine, and where the mutant is substantially insensitive to APP. By insensitive or deregulated is intended that production rates of the mutant are unaffected at concentrations in a nutrient medium which would reduce production rates by at least fifty percent. In addition, the mutant is substantially resistant to catabolite repression as evidenced by lack of response to deoxyglucose at levels which repress respiration in wild-type Candida flareri.

The method involves a course of mutagenesis and fusion, where at each stage, screening is carried out as to enhancement of riboflavin production, associated with particular characteristics related to the purine and riboflavin metabolic pathway. Desirably, the mutants provide for high riboflavin yields in the presence of an amino acid source in the nutrient medium.

The mutagenesis is carried out employing either physical or chemical mutagens with the organisms being grown in a medium stimulating flavinogenesis. Additives which support flavinogenesis include glycine at levels of from about 0.1 to 0.3%, particularly about 0.2 to 0.25%. In addition, trace amounts of cobaltic and zinc ions are desirable. The cobalt concentration will generally be in the range of about 0.5 to 2ag/ml, preferably about 1 µg/ml, while the zinc concentration will generally be about 5 to 25αg/rnl, usually about 5 to 15µg/ml. Optionally Fe⁺³ may be present in from about 0.1-0.3ug/ml. In addition, a carbon source is employed, most sugars being acceptable, e.g., glucose, sucrose, fructose, galactose, etc. With sucrose, the concentration will be generally from about 1 to 8%, preferably about 6%. Comparable concentrations may be used with the other sugars.

At various stages of the mutagenesis and fusion regimen, the mutants will be screened on either defined medium (flavinogenesis-stimulating and lacking amino acids except for glycine) or complex medium (rich nutrient medium containing amino acids), which may contain one or more organic compounds which inhibit one or more enzymes in the purine biosynthesis pathway. Of particular interest is resistance to AMP and APP. In addition, intermediate mutants may be selected on the basis of deregulation of the purine biosynthesis pathway, particularly in the early half of the pathway, more particularly in the first three or four steps.

The resulting mutagen will have at least a 20% increase in riboflavin production over the immediately preceding parent strain and the final mutant will have at least about a 5-fold, preferably at least about a 10-fold, increase in riboflavin production over the original parent strain and have a reduced sensitivity to iron.

Mutagenesis may be carried out either by physical or chemical means, such as irradiation with ultraviolet light or X-rays, or by chemical mutagens, such as nitroso compounds, e.g., nitrosoguanidine, alkylating compounds, e.g., ethyl methanesulfonate, mustard compounds, hydrazine, nitrous acid, or the like. The particular technique for mutagenesis is not critical, and conventional techniques may be employed. The conditions for mutagenesis will generally result in a reduction in viability.

The resultant mutant strains may then be screened for resistance to APP or deoxyglucose, loss of regulation in the purine pathway, or loss of inhibition of B₂ synthesis by iron, AMP and/or a complex medium (includes amino acids, individually or as peptides).

For fusion, protoplasts are employed where the cell wall is removed. A mixture of zymolase, particularly from Arthrobacter luteus, and _{;}8- glucuronidase, particularly from Helix pomatia, is found to be effective. Desirably, the mutant strains are selected as auxotrophs, where each of the strains employed lacks one or more metabolic pathways for production of one or more amino acids. The heterokaryons which are formed may then be selected as prototrophic and screened for enhanced riboflavin production.

At each stage of mutagenesis or fusion, at least a 20%, preferably at least about a 50%, increase in riboflavin production is observed over the original parent. The number of stages will be at least three, and preferably not more than ten, preferably not more than about eight, of strain modifications, involving combinations of mutagenesis and fusion.

The mutagenesis may be carried out in any medium, e.g. a flavinogenesis-stimulating medium, and the resulting viable mutagens screened in a flavinogenesis-stimulating medium. In accordance with the subject invention, mutants are obtained employing different mutagenic agents. One strain is selected from each mutagenesis, one based on enhanced riboflavin production in the presence of adenosine monophosphate and the other strain selected based on enhanced riboflavin production and lack of regulation in an early stage of the purine biosynthesis pathway.

Each of the strains is then marked for amino acid auxotrophy by determining colonies which do not grow on medium lacking a particular amino acid and would grow on medium containing the particular amino acid. In the subject invention, the amino acids selected for were histidine and methionine. Auxotrophs of each of the two strains are treated with saccharidases, particularly a mixture of zymolase and {3-glucuronidase, to prepare protoplasts which are fused in the presence of a nonionic detergent, followed by transfer to a nutrient medium lacking any amino acid source. The resulting prototrophs are examined for riboflavin production and those having enhanced riboflavin production are selected.

The fused cells are then subjected to a further mutagenesis regimen, in this instance chemical mutagenesis, and strains selected for enhanced riboflavin production.

Two strains were isolated of particular interest, designated GA18Y8-6#2 dgr, ATCC Accession No. 20756, and GA18Y8-6#2#11, ATCC Accession No. 20755. In defined medium, these strains produce greater than about 5 grams riboflavin per liter in 6 days, usually greater than about 7 grams per liter in 6 days, employing 5ml of an exponentially growing culture as an inoculum into 50ml of the defined medium. (The medium is 4B + 0.2% glycine + Co + Zn + Fe + 10% YMG (2% glucose, yeast extract, peptone and malt extract). Every 24 hr, the culture is fed 2ml of 10X 4B + glycine + Co + Zn.)

### EXPERIMENTAL

### Mutagenesis

Mutants were obtained by mutagenesis, using either methyl N'-nitrosoguanidine (NTG) or ultraviolet light. The initial parent strain of C. flareri was mutagenized using NTG. The mutant GR3 was obtained using this method on a first culture of the initial parent strain of C. flareri NRRL Y245. 20 ml of an overnight culture of the parent strain was washed and suspended in 20 ml of 4B medium ("defined medium") with 0.2% glycine added. (Burkholder, Proc. Natl. Acad. Sci. USA (1943) 29:166.) (4B medium plus glycine is prepared by dissolving in 1 L water 0.5g KH₂ P0₄; 0.2g MgSO₄ • 7H₂0, 1.84g urea, 60g sucrose, 1µg D-biotin, 20µg H₃BO₄, 20µg MnS0₄, 140µg ZnS0₄, 20µg CuSO₄, 20µg Na₂ Mo0₄ and 2.3g glycine. When cobalt and zinc are indicated 1µg CoCl₂and 10mg ZnC1₂ are added.) NTG (10 mg/ml) in DMSO was added to the culture medium to a final concentration of 100 µg/ml. The culture was stirred at 30 °C for one hour before harvesting and washing.

This treatment resulted in a logarithmic decrease in viable cells.

Mutagenesis was also induced using ultraviolet radiation. The mutant A22 was obtained using this method. A second overnight culture of the parent strain, grown in 4B medium (See, Levine, H., et al., Industrial and Chemical Engineering (1949) 41:1666) with 0.2% glycine, was washed and suspended in an equal volume (35 ml) of the same medium in a plastic petri dish. The medium was then stirred and exposed to ultraviolet radiation of 200-290 nm. This level of irradiation reduced the viable number of cells to 10% of the starting culture. The cells were then plated on defined medium containing 25 mM 5'-AMP. Mutagenesis, plating and incubation were done in the dark. Mutant A22 was found to produce riboflavin in the presence of 5'-AMP, an inhibitor of flavinogenesis.

### Screening Protocol for GR3 Strain

The above-described NTG mutagenized culture was washed twice in 20 ml of saline solution and suspended in the same volume of YMG medium (complex medium). The components of YMG medium are per L, 3 g yeast extract, 3 g malt extract, 5 g peptone, and 100 ml of a carbon source, e.g. glucose, (2%) immediately prior to use. The culture was then incubated with shaking at 30 ° C for 48 hours. The cells were then harvested, washed three times in 20 ml of saline solution and resuspended in 4B medium without urea or glycine. After overnight nitrogen starvation, the cells were harvested and suspended in a yeast nitrogen base medium without amino acids and containing 2% glucose. The culture was then incubated for six hours.

A fresh nystatin solution was prepared by dissolving 1 mg of nystatin in 1 ml of ethanol, followed by dilution to 10 ml with distilled water. The nystatin solution was then added to a final concentration of 20 µg/ml to kill vegetative cells.

Following the nystatin treatment, the viable cells were diluted to 10⁻⁴-10⁻⁵ in saline and 0.1 ml samples were plated onto YMG plates (20 g agar/L of YMG medium). After two days of incubation, the colonies on the YMG plates were replica-plated onto defined medium.

Colonies which grew on the complex medium but not on the defined medium were then streaked onto a defined medium containing adenine at 30 µg/ml. One colony was detected which grew on minimal medium as a white colony, and a pinkish- red colony on the minimal medium with adenine. This isolate, named GR3, was found to produce red colonies in the presence of 20-50 µg/ml adenine and white colonies in the presence of 0 or 100 µg/ml adenine. The red color of the colonies was attributed to the accumulation of P-ribosylaminoimidazole carboxylate (CAIR) and/or P-ribosylaminoimidazole (AIR). It was concluded that GR3 was a mutant defective in the regulation of the first step in the purine pathway. In this GR3 mutant, regulation at the first step is apparently defective because even in the presence of 20-50 µg/ml adenine, purine intermediates accumulated even though the mutant is not auxotrophic for purines.

### Fusions

The GA18 strain was obtained by protoplast fusion of the mutant strains A22 and GR3. Following nystatin treatment as described above, each of the strains diluted in saline (10⁻⁴-10⁻⁵) were plated onto complex medium. After two days incubation, the cells were replica-plated onto 4B medium. Colonies which did not grow on 4B were then streaked onto 4B supplemented either with histidine or methionine at 30 µg/ml. Cells which did not grow on the defined medium but which did grow on the medium supplemented with either amino acid were presumed to be auxotrophs. In this manner, a histidine auxotroph of A22 (A22 His-) and a methionine auxotroph of GR3 (GR3 Met-) were obtained.

To carry out the fusions, 48 hour cultures of A22 His- and GR3 Met- were grown on 4B medium plus glycine, harvested, washed once in 20 ml of distilled water, and suspended in 20 ml of a 1 M sorbitol, 25 mM EDTA and 50 mM dithiothreitol solution. The suspensions were incubated for 20 min at 30 ° C with occasional gentle shaking. The cells were then harvested, washed once in 20 ml of 2 M sorbitol, and each auxotroph was suspended in 20 ml of a 1.5 M sorbitol, 0.1 M sodium citrate, pH 5.8, and 0.01 M EDTA solution. To each suspension was then added 0.2 ml of β-glucuronidase (Type H-2, from Helix pomatia, 100,000 units/ml, Sigma) and 20 mg of zymolase 100T (Miles Laboratory). The suspensions were incubated at room temperature for 45 min. During this time, the normally elongated cells took on a rounded spherical form indicating digestion of the cell walls.

After the enzyme treatment, the protoplasts of each auxotroph were harvested by low speed centrifugation (5000 x g), washed two times in 10 ml 2 M sorbitol, once in 10 ml of 1.5 M sorbitol, 10 mM CaC1₂, and 10 mM Tris HCI pH 7.5, and then suspended in 0.5 ml of the same. To fuse the protoplasts, 0.1 ml samples of each auxotroph were mixed, and 0.1 ml of a solution of 20% PEG (Sigma, approximate molecular weight 6000), 10 mM CaCI₂, and 10 mM Tris HCI pH 7.4 was added.

The mixture was incubated at room temperature for 20 min with occasional shaking. The cells were then centrifuged, washed one time in 10 ml of yeast nitrogen base medium without amino acids with 2% glucose and 1.5 M sorbitol, and suspended in 0.5 ml of the same. Samples of about 0.1 ml, were then added to 7 ml of the same medium containing 2.0% agar (at about 55 °C) and overlaid onto the same medium. Most of the solutions used in this procedure can be found in Methods in Yeast Genetics, Cold Spring Harbor Laboratory, Cold Spring Harbor, New York, 1981, p. 115. In the absence of amino acids, neither auxotroph will grow (there being a very low revertant frequency). Colonies which formed after 6-9 days incubation at 30 ° C contained prototrophs which arose as a result of genetic exchange. Prototrophs resulting from the fusion procedure occurred at a frequency of about one in 10³ survivors of the polyethylene glycol treatment. The colonies were examined under low magnification, and those colonies containing riboflavin crystals were restreaked and examined for riboflavin production in the defined medium. One colony was designated GA18.

### Second Mutation and Screening Yields GA18Y8-6#2

The strain GA18Y8-6#2 was obtained by mutagenizing a 48 hour parent culture of GA18 with the NTG method previously described. Following mutagenesis, the cells were washed, diluted to 10-⁴ and 10-⁵, and 0.1 ml plated on YMG. GA18 did not form yellow colonies, that is, colonies with a high riboflavin content on the YMG medium. This was attributed to repression of flavinogenesis by iron or some organic component in the medium. After a one week incubation period, a few yellow colonies were noted, picked and restreaked several times on YMG to purify the strain. The purified strain was designated GA18Y8-6#2.

### The Mutated Strains

The strains which presently are the most efficient riboflavin producers are identified as Candida flareri GA18Y8-6#2 dgr, having A.T.C.C. Accession No. 20756, and Candida flareri GA18Y8-6#2#11, having A.T.C.C. Accession No. 20755. These strains were derived from the above-described experimental method.

GA18Y8-6#2 dgr produces 7.0 to 7.5 grams riboflavin per liter per 6 days using 5 ml of an exponentially growing culture as an inoculum into 50 ml of the defined medium described previously and supplemented with 0.2µg FeC1₃ and 10% YMG. This strain was obtained by NTG mutagenesis of the parent strain GA18Y8-6#2. Deoxyglucose is an analog of glucose and strongly inhibits cell growth when sucrose is used as a carbon source. Yellow colonies resistant to deoxyglucose were picked, purified by restreaking onto 4B plus glycine and deoxyglucose and tested for flavinogenesis. The mutagenized cells were plated onto 4B plus glycine medium containing 750 µg/ml 2-deoxyglucose. Candida flareri GA18Y8-6#2 dgr was selected from the purified colonies.

GA18Y8-6#2#11 also produces 7.0-7.5 grams riboflavin per liter per 6 days using the same conditions as described previously for GA18Y8-6#2 dgr. This strain was obtained also by NTG mutagenesis of the parent strain GA18Y8-6#2. Following mutagenesis, the 20 ml of mutagenized culture was washed twice in 20 ml of distilled water, then suspended in 20 ml of YMG + FeCl₃ -(2 µg/ml) + AMP (40 mM) + aminopyrazolopyrimidine, APP (500 µg/ml). This medium resulted in the selection of APP resistant colonies. This culture was incubated overnight, with periodic shaking, at 30 °C. The cells were then harvested, washed twice in 20 ml of distilled water, and plated onto YMG + FeC1₃ (2 µg/ml) + APP (1 mg/ml) + AMP (40 mM) and incubated at 30 ° C for a week. At the end of this time, the plates were inspected, and the yellow-orange colonies picked and examined for flavinogenesis. GA18Y8-6#2#11 was obtained from the purified colonies.

High riboflavin yields from the above strains were obtained with 50 ml of a growth medium in a 500 ml baffled growth flask. The growth medium consisted of 10% YMG + 90% 4B + glycine + Co + Zn + FeC1₃ (0.2 µg/ml). Optimal riboflavin production by derivatives of GA18 occurs in the presence of FeCl₃ at 0.2 µg/ml. In the wild type C. flareri, FeCl₃ at 0.2µg/ml represses riboflavin synthesis. The cultures were fed 2 ml of a 10x 4B + glycine solution every 24 hours.

Further enhancement of riboflavin production and iron insensitivity may be achieved by repetition of the mutagenesis, selection and fusion procedures described above.

In accordance with the subject invention inefficient riboflavin producing strains of Candida can be mutated so as to stably produce high levels of riboflavin with efficient utilization of nutrients and improved resistance to iron repression of flavinogenesis.

The foregoing is a complete description of the invention, but is not intended to limit the scope of the invention.

## Claims

1. A method for the production of riboflavin, which comprises:
growing a Candida flareri mutagenized strain in a flavinogenesis-stimulating medium, whereby riboflavin is produced in enhanced amounts,
wherein said C. flareri mutagenized strain is as a result of a regimen of mutagenesis by physical or chemical means and protoplast fusion, wherein after each mutagenesis or fusion, strains are screened in a flavinogenesis-stimulating medium for resistance to an inhibitor of the purine biosynthesis pathway or deregulation of the pruine biosynthesis pathway and wherein said strains and mutants thereof produce at least about 5 grams of riboflavin per litre of fermentation medium in six days.

2. A method according to Claim 1, wherein said resistance is to adenosine monophosphate and/or APP.

3. A method according to Claim 1 or Claim 2, wherein said flavinogenesis-stimulating medium includes stimulating amounts of glycine, cobalt and zinc.

4. A method for the efficient production of riboflavin, which comprises:
growing a Candida flareri mutagenized strain in a flavinogenesis stimulating medium, whereby riboflavin is produced in at least a 5- fold increase over the original parent strain;
wherein said C. flareri mutagenized strain is as a result of separately mutagenizing said parent strain with two different mutagens to provide mutagenized strains;
first screening in a flavinogenesis stimulating medium for enhanced riboflavin production and a change in the regulation of the purine biosynthesis pathway or response to inhibitors of the purine biosynthesis pathway, to provide first and second derived strains which result from different mutagenic agents;
preparing protoplasts from the first and second derived strains and fusing said protoplasts to provide fusion strains;
second screening in a flavinogenesis stimulating medium said fusion strains for at least enhanced riboflavin production to provide a third derived strain;
mutagenizing the third derived strain to provide second mutagens;
third screening in a flavinogenesis stimulating medium said second mutagens for at least enhanced riboflavin production to provide a fourth derived strain; and
repeating said mutagenizing and screening as necessary to obtain a strain having at least a 5-fold increase in riboflavin production.

5. A method according to Claim 4, wherein:
said screenings include selecting at least once for: resistance to adenosine monophosphate; resistance to 4-amino pyrazolo-(3,4-d)pyrimidine; and resistance to iron.

6. A method for the production of riboflavin, which comprises:
growing a Candida flareri mutagenized strain in a flavogenesis-stimulating medium, said strain capable of producing at least about 5 grams of riboflavin per liter of fermentation medium in six days, wherein said strain is resistant to inhibition of B₂ synthesis by adenosine monophosphate, resistant to inhibition of growth by 4-aminopyrazolo-(3,4-3)pyrimidine and resistant to catabolite repression in the presence of deoxyglucose when sucrose is the carbon source.

7. A method according to claim 6, wherein said strain is resistant to inhibition of B₂ synthesis by iron or complex medium.

8. A method according to claim 6, wherein said strain produces at least about 5 grams of riboflavin per liter in six days.

9. A method according to claim 6, wherein said strain is GA18Y8-6#2 dgr, ATCC Accession No. 20756 or GA18Y8-6#2#11, ATCC Accession No. 20755, or strains derived therefrom.

## Patentansprüche

1. Verfahren zur Erzeugung von Riboflavin, welches umfaßt:
das Züchten eines mutierten Candida flareri-Stamms in einem Flavinogenese stimulierenden Medium, wodurch Riboflavin in erhöhten Mengen erzeugt wird,
worin der genannte mutierte C.flareri-Stamm ein Ergebnis eines Mutageneseschemas durch physikalische oder chemische Mittel und Protoplastfusion ist, worin nach jeder Mutagenese oder Fusion Stämme in einem Flavinogenese stimulierenden Medium auf Resistenz gegen einen Inhibitor des Purinbiosynthesewegs oder Deregulierung des Purinbiosynthesewegs gescreent werden, und worin die genannten Stämme und Mutanten davon in sechs Tagen zumindest etwa 5 g Riboflavin pro Liter Fermentationsmedium erzeugen.

2. Verfahren nach Anspruch 1, worin die genannte Resistenz eine gegen Adenosinmonophosphat und/oder APP ist.

3. Verfahren nach Anspruch 1 oder 2, worin das genannte Flavinogenese stimulierende Medium stimulierende Mengen Glycin, Kobalt und Zink einschließt.

4. Verfahren zur wirksamen Erzeugung von Riboflavin, welches umfaßt:
das Züchten eines mutierten Candida flareri-Stammes in einem Flavinogenese stimulierenden Medium, wodurch Riboflavin mit zumindest 5-facher Steigerung gegenüber dem ursprünglichen Elternstamm erzeugt wird;
worin der genannte mutierte C.flareri-Stamm ein Ergebnis des getrennten Mutierens des genannten Elternstamms mit zwei verschiedenen Mutagenen ist, um mutierte Stämme zu erzeugen;
erstes Screenen in einem Flavinogenese stimulierenden Medium für verstärkte Riboflavinproduktion und eine Veränderung der Regulierung des Purinbiosynthesewegs oder Reaktion auf Inhibitoren des Purinbiosynthesewegs, um erste und zweite abgeleitete Stämme zu schaffen, die aus verschiedenen mutagenen Mitteln resultieren;
Herstellen von Protoplasten aus den ersten und zweiten abgeleiteten Stämmen und Fusion der genannten Protoplasten, um Fusionsstämme zu schaffen;
zweites Screenen der genannten Fusionsstämme in einem Flavinogenese stimulierenden Medium, für zumindest verstärkte Riboflavinproduktion, um einen dritten abgeleiteten Stamm zu schaffen;
Mutieren des dritten abgeleiteten Stamms, um zweite Mutagene zu schaffen;
drittes Screenen der genannten zweiten Mutagene in einem Flavinogenese stimulierenden Medium für zumindest verstärkte Riboflavinproduktion, um einen vierten abgeleiteten Stamm zu schaffen; und
Wiederholen des genannten Mutierens und Screenens wie erforderlich, um einen Stamm zu erhalten, der zumindest eine 5-fache Steigerung der Riboflavinproduktion aufweist.

5. Verfahren nach Anspruch 4, worin die genannten Screeningvorgänge das zumindest einmalige Auswählen für: Resistenz gegen Adenosinmonophosphat; Resistenz gegen 4-Aminopyrazol(3,4-d)pyrimidin; und Resistenz gegen Eisen einschließen.

6. Verfahren zur Erzeugung von Riboflavin, welches umfaßt:
das Züchten eines mutierten Candida flareri-Stamms in einem Flavinogenese stimulierenden Medium, wobei der genannte Stamm fähig ist, in sechs Tagen zumindest etwa 5 g Riboflavin pro Liter Fermentationsmedium zu erzeugen, worin der genannte Stamm gegen die Hemmung von B₂-Synthese durch Adenosinmonophosphat resistent ist, gegen die Hemmung von Wachstum durch 4-Aminopyrazol-(3,4-d)pyrimidin resistent ist und gegen Katabolitrepression in der Gegenwart von Desoxyglukose resistent ist, wenn Saccharose die Kohlenstoffquelle ist.

7. Verfahren nach Anspruch 6, worin der genannte Stamm gegen die Hemmung von B₂-Synthese durch Eisen- oder Komplexmedium resistent ist.

8. Verfahren nach Anspruch 6, worin der genannte Stamm in sechs Tagen zumindest etwa 5 g Riboflavin pro Liter erzeugt.

9. Verfahren nach Anspruch 6, worin der genannte Stamm GA18Y8-6#2 dgr, ATCC-Zugangsnummer 20756, oder GA18Y8-6#2#11, ATCC-Zugangsnummer 20755, oder davon abgeleitete Stämme ist.

## Revendications

1. Méthode de production de riboflavine, qui comprend :
la croissance d'une souche mutagénisée de Candida flareri dans un milieu stimulant la flavinogénèse, pour ainsi produire la riboflavine en quantités améliorées,
où ladite souche mutagénisée de C. flareri est le résultat d'un régime de mutagénèse par des moyens physiques ou chimiques et la fusion des protoplastes,
où, après chaque mutagénèse ou fusion, les souches sont examinées, dans un milieu stimulant la flavinogénèse ,
pour leur résistance à un inhibiteur du trajet de la biosynthèse de la purine ou la dérégulation du trajet de la biosynthèse de la purine et où lesdites souches et leurs mutants produisent au moins environ 5 grammes de riboflavine par litre du milieu de fermentation en six jours.

2. Méthode selon la revendication 1, où ladite résistance est vis-à-vis de l'adénosine monophosphate et/ou APP.

3. Méthode selon la revendication 1 ou la revendication 2, où ledit milieu stimulant la flavinogénèse contient des quantités stimulantes de glycine, cobalt et zinc.

4. Méthode pour la production efficace de riboflavine qui comprend :
la croissance d'une souche mutagénisée de Candida flareri dans un milieu stimulant la flavinogénèse,
ainsi la riboflavine est produite à une augmentation d'au moins 5 fois par rapport à la souche parente d'origine ;
où ladite souche mutagénisée de C. flareri est le résultat de la mutagénèse séparée de ladite souche parente avec deux mutagènes différents pour donner des souches mutagénisées ;
un premier examen, dans un milieu stimulant la flavinogénèse,de la production améliorée de riboflavine et d'un changement de la régulation du trajet de la biosynthèse de la purine ou de la réponse à des inhibiteurs du trajet de la biosynthèse de la purine, afin de produire des première et seconde souches dérivées résultant de différents agents mutagènes ;
la préparation de protoplastes à partir des première et seconde souches dérivées et la fusion desdits protoplastes pour donner des souches de fusion ;
un second examen,dans un milieu stimulant la flavinogénèse, desdites souches de fusion pour une production au moins améliorée de riboflavine afin de produire une troisième souche dérivée ;
la mutagénèse de la troisième souche dérivée pour produire des seconds mutagènes ;
un troisième examen, dans un milieu stimulant la flavinogénèse,desdits seconds mutagènes pour une production au moins améliorée de riboflavine afin de produire une quatrième souche dérivée; et
la répétition de la mutagénèse et de l'examen selon ce qui est nécessaire pour obtenir une souche ayant une augmentation d'au moins 5 fois de la production de riboflavine.

5. Méthode selon la revendication 4, où :
lesdits examens comprennent la sélection au moins 1 fois pour: la résistance à l'adénosine monophosphate ; la résistance à la 4-amino pyrazolo(3,4-d)pyrimidine ; et la résistance au fer.

6. Méthode pour la production de riboflavine, qui comprend :
la croissance d'une souche mutagénisée de Candida flareri dans un milieu stimulant la fla- vogénèse, ladite souche étant capable de produire au moins environ 5 grammes de riboflavine par litre du milieu de fermentation en six jours, où ladite souche résiste à l'inhibition de la synthèse de B₂ par l'adénosine monophosphate, résiste à l'inhibition de la croissance par la 4-aminopyrazolo-(3,4-3)pyrimidine et résiste à la répression des catabolites en présence du désoxyglucose quand le saccharose est la source de carbone.

7. Méthode selon la revendication 6, où ladite souche est résistante à l'inhibition de la synthèse de B₂ par le fer ou un milieu complexe.

8. Méthode selon la revendication 6, où ladite souche produit au moins environ 5 grammes de riboflavine par litre en six jours.

9. Méthode selon la revendication 6, où ladite souche est GA18Y8-6#2 dgr, Numéro d'Accession ATCC 20756 ou GA18Y8-6#2 #11, Numéro d'Accession ATCC 20755 ou des souches qui en sont dérivées.
